# EUROPEAN PATENT SPECIFICATION

(11) **EP 1 124 440 B1**
(45) Date of publication and mention of the grant of the patent: **13.04.2005**
(21) Application number: 99951026.6
(22) Date of filing: 29.10.1999
(51) Int. Cl.: A23L 1/00

(54) **USE OF CALCAREOUS MATERIAL FOR FOODSTUFF AND COSMETIC MATERIAL**
VERWENDUNG VON KALKMATERIAL FÜR LEBENSMITTEL UND KOSMETIKA
COMPOSITIONS DE PRODUITS ALIMENTAIRES

(30) Priority: 30.10.1998 GB 9823885
(43) Date of publication of application: 22.08.2001
(73) Proprietor: Aquacal Limited, Carrigaline, County Cork (IE)
(72) Inventor: Assoumani, Mohamed Bakri, Carrigaline, County Cork (IE)
(74) Representative: Doble, Richard George Vivian
(86) International application number: PCT/GB1999/003580
(87) International publication number: WO 2000/025602

(56) References cited:
- WO-A-98/33508
- BE-A- 693 094
- DE-A- 2 947 186
- FR-A- 2 201 040
- DATABASE WPI Section Ch, Week 199314 Derwent Publications Ltd., London, GB; Class D13, AN 1993-111855 XP002132742 & JP 05 049446 A (KANKYO HOZEN KENKYUSHO YG), 2 March 1993 (1993-03-02)

## Description

This invention relates to solid and semi-solid foodstuff compositions particularly foodstuff compositions containing calcium materials.

The fortification of foodstuffs with calcium compounds is well recognised as a means of supplementing calcium in the diet. However, the addition of presently available sources of calcium has been found to result in deterioration in the physical properties of the product to which calcium is added when added in the amounts sufficient to give the desired available calcium so as to ensure an adequate intake of calcium in the diet.

In International Patent Application no. PCT/GB98/00142 published as WO/98/33508 there is disclosed the use of a very pure form of corallinaceae for treatment of conditions created by failure of immuno regulation in the body. This has included the use of corallinaceae for the manufacture of a medicament for the treatment of reduced calcium levels and use in manufacturing medicament for raising pH levels in the colon. This application discloses forming emulsions in the manufacture of foods wherein an emulsifier is combined with a residue of a very pure form of corallinaceae (Maërl) and then with an oil phase of a foodstuff which is formed into an emulsion with an aqueous phase. There is reference to the use of these oil products in bakery products. However, this specification relates primarily to inclusion of the residues for nutritive purposes and does not indicate generally the value of this particular material in relation to starch products particularly farinaceous products nor does it discuss improvements in physical, including organoleptic, properties.

Studies of the addition of corallinaceae by-products and residues to foodstuffs have been in relation to nutritive properties (Agro-food-Industry Hi-Tech; September/October, 1997 and see a subsequent article in the September-October 1998 issue). These articles have discussed the properties of calcareous materials in terms of bioavailability of calcium. The high surface area of corallinaceae products appeared to correlate with solubility at various pH's which correlated with calcium absorption and with physiological and biochemical properties arising from such bioavailability. Similarly the later article refers to buffering and similar properties and discusses anti-acid properties, mentions acid uptake in the context of organic juice and particularly structure, texture and mouthfeel in that connection, ie. of beverage.

It has now been found that if a form of corrallinaceae is employed in the manufacture of solid and semi-solid foodstuffs much superior results are obtained in the texcure of the resulting product. In particular it is possible to add higher amounts of calcium than is possible with other sources of calcium.

Without limitation on the invention, it is believed that the advantages of the invention, at least partially, arise from the unusual structure of the calcareous material employed. It is believed that the calcareous material has a porous structure which on hydration or oil absorption collapses to give a very smooth structure. This is analogous to a hydrocolloid or edible gel which holds the fluid phase in extremely small pores. Structure collapse can be achieved with an amount of moisture of 2% by weight.

Other properties of the calcareous material are film forming properties, adhesiveness and binding properties and non-abrasiveness. These properties are not found in known food grade calcium carbonate materials. These properties were not appreciated from the previous work on the calcareous material based on the invention which was primarily directed to the nutritive and pharmacological properties.

The absorption capacity and binding properties can be of particular advantage in cereal products and may explain the positive effects on stickiness and strength. These effects also assist in applications of carbohydrates (sugars) for example sugar (eg. sucrose or fructose), syrups and honey. There is reduction of water activity and extension of shelf life and improvement of flavour formation by non-enzymatic browning.

The calcareous material does not require prior solubilisation for use in semi-moist or dry products. At about pH 6.5, approximately 19% by weight of the calcium content will ionise and the carbonate portion will slightly increase pH and buffer the system. It is believed this will contribute to homogenous browning (Maillard reactions (with possible Strecker degradations)) during a cooking stage and better flavour formation. These give rise to unexpected advantages in texture, colour, flavour and shelf-life.

The invention therefore has two aspects. The first is the improvement in fatty products where the calcareous material is in the fatty phase and enhances emulsion stability, controls fat crystallisation and enhances organoleptic properties. Incidentally this permits inclusion of sufficient calcareous material to allow incorporation of calcareous material to give in excess of 25% of ERDA requirements.

The second is the improvement as discussed subsequently on non-fatty products of physical and organoleptic properties.

This has been found to be of particular application to solid or semi-solid products as distinct from beverages. By a solid or semi-solid product is meant one having significant shape-retaining properties as distinct from flowable liquid compositions which have low or non-existent shape retaining properties so that they would normally be classified as beverages.

While the improvements of the invention can be obtained in high fat compositions, for example emulsified fat products in which the calcareous material is incorporated in an oil phase, improvements are achieved in non-emulsified compositions, for example cheese spreads and yoghurt-type products. Improvement is also found in fatty and non-fatty products of the ice-cream type, ie. intended for consumption while still frozen.

As will be described in detail, a significant improvement is found in farinaceous products when the calcareous materials of the invention are added.

Produces of the invention also include confectionery particularly carbohydrate products, ie. products consisting to a significant extent of sugars such as sucrose. These can include candy products, gelatinous products and particularly chocolate based products including cocoa fat products and other fat products and chocolate products such as cocoa. Candy products can be boiled sugar products and other confectionery products. Confectionery products include dessert products including pudding mixes and gelatinous products. The invention also applies to meat derived products.

The calcareous material used in the invencion provides calcium in carbonate form. It contains 34, by weight calcium as compared to 40% by weight for conventional commercial calcium carbonate.

By adding the calcareous material according to the invention it is possible to achieve a known RDI (Recommended Dietary Intake) for a specified weight or volume of foodstuff and also, generally, improve the qualities of the product, for example texture, mouthfeel, strength and cooking properties.

A calcareous material useable in the invention is more fully described in the above international patent application. The calcareous material is obtained from corallinaceae.

Coralinaceae, for example Lithothamnium corallicides (Lithothamnium calcareum sometimes known as phymatolithon calcareum), are known seaweeds which are very abundant in certain cold and temperate seas. Once harvested the crude residual product consists primarily of mineral substances, particularly calcium carbonate and magnesium carbonate. The largest component is calcium carbonate, often about 34% by weight. This produce is sometimes identified as Maërl though the term Maërl encompasses residues of coralline algae of various members of the order corallinales (Class Rhodophyceae) including members of corallinaceae for example members of the species Lithothamnium corallioides, Phymatolithon calcareum and Lithothamnium glaciale.

Crude corallinaceae (Lithothamnium corallioides) residues have been commercially available for use in the prevention of acidosis in intensively fed cows. In French patent FP2 201 040 there is disclosed the use of Maërl which appears to be in the crude form for animal feeds. Such products as have been available until the present time have tended to be relatively impure products frequently from contaminated sources. Usually they contain significant amounts of siliceous materials derived from the original product as dredged and other non-corallinaceae residues for example ground shells of sea-creatures.

In BE 693094A (UK 1113318A) there is disclosed a product of fragmented seaweed cells which seaweed includes Lithothamnium calcareum which is obtained by fragmenting seaweed as it is harvested and drying and purifying. This product is from the whole natural seaweed and is not a calcareous product obtained from the residues of seaweed which contain primarily calcium carbonate so it contains primarily carbonates, particularly calcium carbonate.

The Institute of Oceanography in Paris produced a report on corallinaceae particularly Lithothamnium in 1989 describing the residual crude product (Maërl) and describing its use in treatment of soil and for animal feed as a dietary supplement and for treatment of water.

Corallinaceae particularly Lithothamnium corallioides are coralline algae. There are a number of sub-species of corallinaceae particularly Lithothamnium differentiated by morphological data but these data can vary depending on local sea bed and weather conditions. Other known "relatives" include Phymatolithon calcareum and in more northerly regions Lithothamnium glaciale. These plants lay down calcium carbonate in their cell walls which gives them a hard stony texture. The living corallinaceae for example Lithothamnium corallioides generally show a red colour due to the presence of a pigment phytoerythrin in their structure. When dead the colour is white or yellowish. Corallinaceae for example Lithothamnium corallioides occurs naturally in cold and temperate seas and has been reported in Norway, Canada, Scotland, Ireland and France.

Since compositions of the subject invention are to be used in foodstuffs it is of course important that the corallinaceae which is to be exploited in the invention is derived from a part of the world which does not suffer from heavy pollution. For this purpose corallinaceae particularly Lithothamnium corallioides harvested from stocks north of Lonehort Point, Castletownbere, County Cork in the Republic of Ireland have proved very satisfactory but there are also deposits off the West Coast of Galway.

Naturally occurring residues of Lithothamnium corallioides were harvested at the above site at Lonehort Point, purified and concentrated.

The raw material can be purified by initial extensive washing with sea and fresh water together with removal of extraneous sand, shells, and other debris particularly siliceous debris such as stones. This step usually reduces the material obtained by dredging from the sea bed to about 20% by weight.

The cleaned and separated product is then subjected to intensive cleaning by for example, bleaching and sterilising in hydrogen peroxide for from 8 to 24 hours, further washing in water, drying in a sterile fluid bed and final milling under bacterial controlled conditions.

For the purpose of this invention it is important for compositions intended for consumption (edible products) that they comply with Food Regulations, for example in relation to the upper limits for contents of heavy metals. This may result inherently from natural source or from the technique of purification.

The stringent washing conditions can reduce sodium content of the raw product from amounts in the order of well in excess of 1,000 ppm for example amounts up to 5,200 ppm to sodium contents in the low hundreds, for example 300 ppm. Thus there can be a reduction of about 10 fold in the sodium content as compared to raw material.

The silica content of this final material is normally not more than 0.5% by weight as compared to a silica content in previously available Maërl from a commercial source, of about 5% by weight.

A representative sample of this purified, concentrate contained the following elements in the following amounts (by weight):

| | |
|---|---|
| Calcium | 34% |
| Magnesium | 2.4% |
| Phosphorous | 0.08% |
| Potassium | 0.10% |
| Sulphur | 0.45% |
| Iron | 25 ppm |
| Boron | 16.5 ppm |
| Fluorine | 200 ppm |
| Sodium | 310 ppm |
| Manganese | 125 ppm |
| Nickel | 30 ppm |
| Cobalt | 6 ppm |
| Copper | 10 ppm |
| Lead | 460 ppb |
| Zinc | 37 ppm |
| Selenium | 1 ppm |
| Molybdenum | 39 ppm |
| Iodine | 160 ppm |
| Arsenic | <1 ppm |
| Chromium | 13 ppm |
| Cadmium | 0.2 ppm |
| Mercury | <50 ppb |
| Aluminium | <5 ppm |

According to the present invention there is provided use in a solid or semi-solid foodstuff of a material to improve organoleptic and physical properties and calcium content characterised in that the material is a calcareous residue of corallinaceae with a content of heavy metals below the upper limits acceptable for edible products.

The calcareous residue can be incorporated in a foodstuff having an emulsified oil or fat phase into which is incorporated the corallinaceae residue and which has improved organoleptic properties as compared to the same product free of said residue but advantages also exist for a foodstuff containing fat material in which the corallinaceae residue is distributed generally in the foodstuff.

Particularly valuable foodstuffs in which the invention has advantage is ones to be consumed in frozen form. The invention is also applicable to yoghurt products.

The invention is also particularly applicable to carbohydrate products including desserts, confectionery and similar products or chocolate products.

In non-fat products the calcareous material is preferably added by a carbohydrate (sugar) water phase.

The foodstuff can contain a sufficient proportion of the calcareous material as defined above derived from corallinaceae to provide a substantial proportion of the Recommended Dietary Intake of calcium in the daily diet. The foodstuff in question is primarily intended for human beings although the invention could be applied to foodstuffs for animals.

Particular foodstuffs are starch based foodstuffs, especially those derived from farinaceous materials i.e. those based primarily on wheat or similar farinaceous grains. Particular products in which the calcareous material can be employed include bread, so-called biscuits or wafers, the various forms of pasta including noodles, breakfast cereals and extruded farinaceous products and so-called snack foods.

Particularly in relation to pasta and as discussed in an article by J Shewing on the Texture of Pasta in Cereal Foods World January 1997 volume 42 no. 1 pages 8 through 12 microstructure changes profoundly affect the properties of the resulting pasta and changes in the components can radically change the hydration characteristics. In that article there are described assessment of various product characteristics both cooked and uncooked pasta products.

The proportion of calcareous product added can depend on the final desired calcium Recommended Dietary Intake or the improvement in physical (eg) organoleptic properties but for example can range up to 4 or 5% by weight of the basic raw materials in forming the final food product. The preferred range is 0.5 to 3% by weight most preferably 1 to 2% by weight more particularly it is up to about 1.6% by weight of the product. For example in biscuits intended to supplement a diet with calcium one can employ approximately 20 grams per so-called biscuit representing about 2% of the final product.

The addition of the calcareous product used in the invention as compared to the results when other sources of calcium are used not only improved the properties of baked products but, in some instances, has also been found to provide a buffering effect in the stomach and also appears to provide anticariogenic effects. It is believed that this may partly arise from protection against acid in the mouth.

The invention will now be illustrated by the following examples which are not however intended to limit the scope of the invention. The Calcium Product (calcareous produce derived from corallinaceae) employed is a commercial product prepared from Lithothamnium coralliodes residues as described above and having the analysis set out above and identified commercially as AquaMin (AquaMin is a trade mark registered in certain jurisdictions). The coated Calcium Product is the calcereous product coated with a mono-diglyceride.

### Example 1

### Fortification of pasta with calcium.

A conventional pasta product of the spaghetti nature manufactured from durum or other hard wheat flour was employed.

### Manufacture of Pasta:

A basic recipe for pasca was used.

Durham Wheat Semolina (766g) + Tap Water (234g) → 1Kg of Pasta.

| |
|---|
| Mix for 10 min in mixing chamber of the pasta press |
| ↓ |
| Rest for 5 min |
| ↓ |
| Warm up die : Extrude |
| ↓ |
| Cook for four min |
| ↓ |
| Cool for 30-60 sec under running tap water |
| ↓ |
| Analyse |

The following batches of conventional pasta were made and analysed.
1. Control (no added Calcium).
2. Pasta + 0.73% Calcareous Product (40% R.D.I./150g serving).
3. Pasta + 1.26% Calcareous Product (70% R.D.I./150g serving).
4. Pasta + 1.4% Coated Calcareous Product (70% R.D.I./150g serving).
5. Pasta + 1.07% Calcium Carbonate control (70% R.D.I./150g serving).

### [R.D.I. - Recomended Dietary Intake]

100g of pasta was then cooked in 500ml of water for four minutes, the pasta was then analysed for firmness and stickiness using a texture analyser (AACC 16-50 standard method).

### Results:

| **Sample** | **Firmness (Force g)** | **Stickiness (Force g)** |
|---|---|---|
| **Batch one** | 333.022 | -1019.63 |
| **Batch two** | 377.902 | -1019.927 |
| **Batch three** | 300.144 | -1018.654 |
| **Batch four** | 310.046 | -1018.367 |
| **Batch five** | 291.144 | -1017.703 |

The control batch and batch two (40% R.D.I.) were made and analysed on the same day. The test results showed that addition of Calcareous Product increased the firmness of the pasta and reduced the stickiness when compared to the control.

At a higher level of Calcareous Product addition, additional water was added (5ml/1 Kg pasta) to prevent the pasta becoming too firm. Therefore a direct comparison cannot be made between batches 1,2 and 3,4,5.

Batch five was significantly stickier than any of the other batches of pasta. This was evident in handling the pasta as strands tended to stick together. This did not happen to the other batches.

The organoleptic qualities - colour, volume, speckledness, glossiness and bulkiness - of each batch of pasta were similar and it was impossible to detect any differences in taste between the batches.

The fortification of fresh pasta with a Calcareous Product as employed in this invention was very successful, increasing the strength of the pasta and reducing the stickiness.

### Example 2

### Fortification of biscuits with Calcium

Four batches of biscuits were made using the following recipe:

| | |
|---|---|
| 400g | Confectionery Flour |
| 166.8g | Fat |
| 140g | Sugar |
| 20g | Syrup |
| 2.8g | Salt |
| 2.0g | Ammonium Bicarbonate |
| 2.0g | SSL (Sodium Stearoyl Lactylate) |
| 73g | Water |

| | |
|---|---|
| 1. | Batch one: Control - no added Calcium. |
| 2. | 1.8% Calcareous Product. |
| 3. | 2.0% Coated Calcareous Product } 40% Calcium R.D.I. per serving* |
| 4. | Calcium Carbonate Control. |

| | |
|---|---|
| * one serving of biscuits is three biscuits (20g in weight each). | |

The biscuits were cooked for exactly eleven minutes and then analysed.

The following parameters were examined: friability, water activity (Aw) and colour.

| **Sample** | **Friability** | **Aw** |
|---|---|---|
| **Batch one** | 3483.41 | 0.306 |
| **Batch two** | 4275.13 | 0.353 |
| **Batch three** | 3406.77 | 0.335 |
| **Batch four** | 1333.66 | 0.520 |

Colour was measured using LAB values.
L. Brightness
A. Red
B. Yellow

| **Sample** | **L value** | **A value** | **B value** |
|---|---|---|---|
| **Batch one** | 60.64 | 10.52 | 33.78 |
| **Batch two** | 63.66 | 9.42 | 31.77 |
| **Batch three** | 63.63 | 9.60 | 31.83 |
| **Batch four** | 72.87 | 3.33 | 33.18 |

### Friability

The results showed that addition of Calcareous Product increased the friability of the biscuit when compared with the control (3483.4-4275.1) and addition of coated Calcareous Product decreased the friability of the biscuit. However these differences could not be detected by a taste panel. The friability of the biscuits fortified with Calcium Carbonate were significantly reduced and this was very obvious to the taste panel who felt the biscuits tasted soft/gone off.

### Water Activity

The water activity of the biscuits fortified with Calcium Carbonate was significantly increased when compared with the control. Addition of either Calcareous Product or coated Calcareous Product did not have a significant effect on the water activity of the biscuits.

### Colour

The LAB values of the biscuits were measured using a Minolta colour meter.

The lightness of the biscuits fortified with Calcareous Product and coated Calcareous Product were marginally increased, whereas the biscuits fortified with Calcium carbonate increased from 60.64-72.87.

The red colour of the biscuits fortified with Calcium Carbonate was significantly reduced when compared with the control, Calcareous Product and coated Calcareous Product had little effect on this parameter.

The yellow colour of the biscuits was marginally reduced in both the biscuits with additional Calcareous Product, Calcium carbonate did not effect this value.

The taste panel were in agreement that there was very little difference in the appearance and taste of the biscuits fortified with Calcareous Product and coated Calcareous Product when compared with the control. Most people were unable to identify which biscuits had the additional Calcium. However the biscuits fortified with Calcium Carbonate were pale in colour, soft and unpalatable to taste (loss of sweet flavour).

### EXAMPLE 3

### CALCIUM FORTIFICATION OF SPREADS

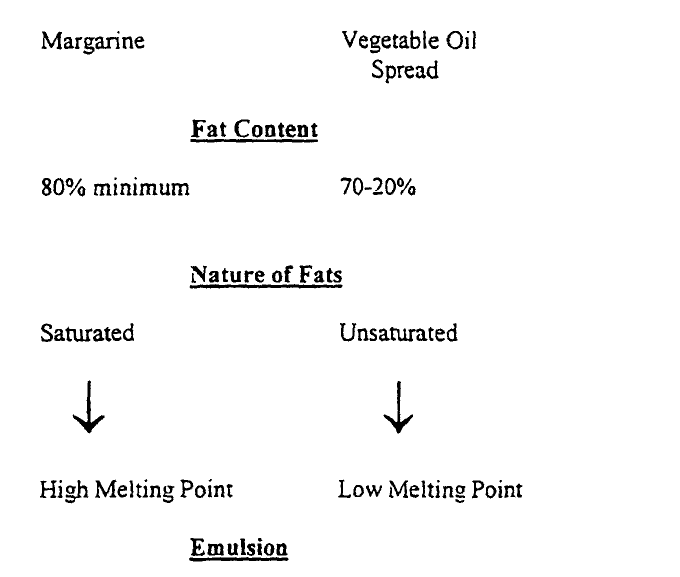

There are two phases :

| | |
|---|---|
| Water Phase | Oil Phase |

The two phases are mixed to build the emulsion. Emulsion building requires energy input in the form of mechanical agitation, ultrasonic vibration or heat.

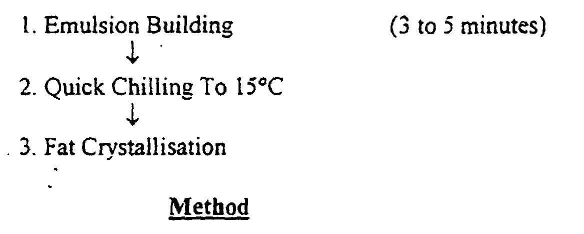

AquaMin must be added to the oil phase : the oil will go inside the pores. This will help stabilise the emulsion.

The order of mixing is critical for the addition of AquaMin to this type of emulsion structure spread.

If AquaMin is added to the water phase first, then the water enters the porous structure and these pores become polar. The outside surface of AquaMin is also polar, so that when this is now mixed with the oil, which is hydrophobic, this will destabilise the emulsion.

If however, AquaMin is added to the oil phase first, then the oil enters the pores and due to the oil viscosity it is retained inside. The oil, being hydrophobic, now makes the internal pores hydrophobic. The outside AquaMin surface area is still polar, so now when added to the water phase, which is also polar, a stable emulsion will result.

After the emulsion building stage, chilling and fat crystallisation follow and during the crystallisation stage, AquaMin promotes the formation of the β' crystal form. This crystal structure is most desirable, as it requires less energy to melt than the larger β form and is more stable than the smaller lower energy α form and consequently, the β' crystals give the spread a better mouth feel. As a result of this, in the spread AquaMin has excellent uniform calcium distribution, with no detection of the presence of particles in the mouth.

The only technical issues to be aware of in terms of the impact on the quality of the finished spread are:
- AquaMin's high buffering capacity may affect the titratable acidity of the spread, so in this case it will be necessary to monitor the titratable acidity during the process and compensate through the addition of lactic acid.
- At addition rates above 2%, the off-white colour of AquaMin may affect the colour of the spread, so here it will be necessary to add beta-carotene to the formulation to counter this.

Both issues are dependent on the level of AquaMin addition and will vary on the composition of the spread in terms of fat content, but are easily overcome using ingredients that are universally used during the production process.

### EXAMPLE 4

### CALCIUM FORTIFICATION OF CHEESE SPREADS

A cheese spread was made using a standard recipe of:
Young, Medium-Ripe and Over-Ripe Cheddar Cheese
Water
Butter
Whey Powder
Emulsifier
Salt
Preservatives

### Method

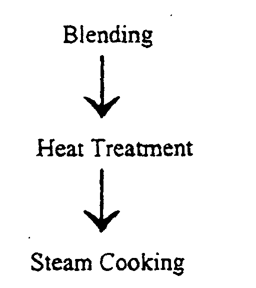

AquaMin is added during the cooking stage and disperses homogeneously throughout the spread. AquaMin can be easily incorporated at levels of 1-2% without any adverse effects. At 2% AquaMin addition in a 200g tub, a 15g serving will provide 12.75% of the RDI for Calcium (the European Union RDI for Calcium is 800mg/day).

### EXAMPLE 5

### CALCIUM FORTIFICATION OF YOGHURT

In the case of yoghurt production, industrial production typically follows the following process:

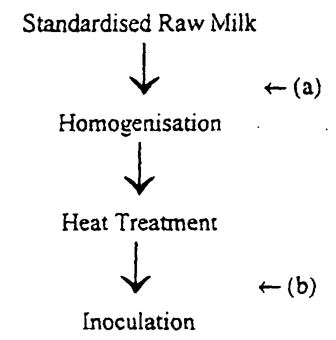

### Method

AquaMin can be added either (a) before homogenisation or (b) after heat treatment. (a) is preferred as calcium ionisation will be improved and will promote the Ca⁺⁺ interaction with denatured αs-casein. This can result in a slight increase in viscosity.

In the case of stirred yoghurt with fruit, AquaMin can be added to the fruit purees before heat treatment. Ca⁺⁺ will help stabilise fruit puree through the formation of calcium pectate.

This aspect of the invention relates to solid or semi-solid yoghurt compositions as distinct from beverages based on yoghurt.

### EXAMPLE 6

### CALCIUM FORTIFICATION OF ICE-CREAM

Ice-cream was made using a standard recipe as follows:

| | |
|---|---|
| Fat | 17% |
| MSNF | 11% |
| Sugar | 14% |
| Emulsifier | 0.5% |
| Water | 57% |

Two batches of ice-cream were made:
1. Control
2. + 0.79% AquaMin
(Addition of AquaMin provides 100% Calcium fortification in a 200g serving)

### Method

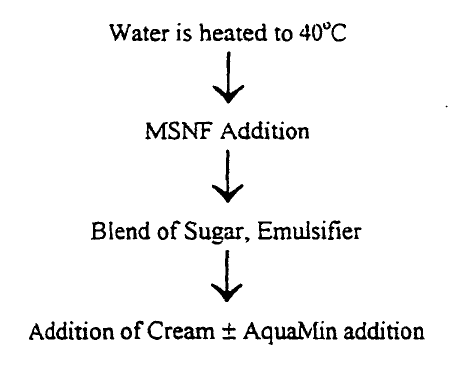

The ice-cream solution is mixed continuously using a Silverson mixer. The solution is then:

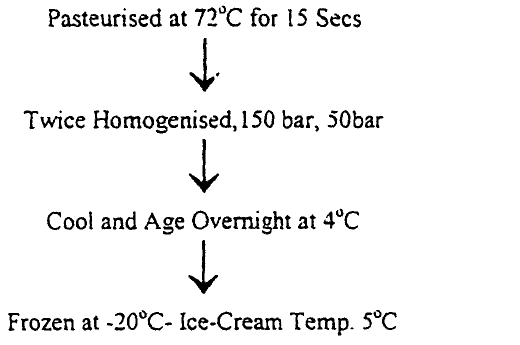

During freezing samples of ice-cream were taken from the different batches at different times to ensure homogenous dispersion.

The following parameters of the ice-cream were examined
1. Calcium Analysis
2. Sensory Analysis
3. Colour
4. Over-Run

1. Calcium analysis confirmed that the calcium was homogeneously dispersed throughout the ice-cream.

| Sample | Observed (PPM) |
|---|---|
| Control | 1670 |
| AquaMin | 4070 |
| Calcium Carbonate | 3950 |

Samples of ice-cream taken at different stages during the production had similar calcium levels
2. A sensory analysis of the ice-cream was carried out in a local university under controlled conditions in their sensory analysis unit (report available upon request). Panellists were asked to assess ice-cream using the following parameters :
Sample:
Taste (1 = very poor, 5 = very good)
Grittiness (1 = very gritty, 5 = not gritty)
Overall acceptability (1 = worst, 5 = best) /..........
19 panellists took part in this analysis and the results were as follows

| Sample | Taste | Grittiness | Acceptability | Preference |
|---|---|---|---|---|
| **A, AquaMin** | 3.2 | 4.6 | 3.6 | 15 |
| **B, Calcium Carbonate** | 2.8 | 4.3 | 2.9 | 1 |
| **C, Control** | 2.7 | 2.9 | 2.3 | 3 |

From the results it is clear that AquaMin fortified ice-cream is predominant -79% of panellists preferred the ice-cream fortified with AquaMin. The control and the ice-cream fortified with calcium carbonate lagged behind, with only 16% and 5% of preferences respectively. The ice-cream fortified with AquaMin scored highest on all parameters of taste, grittiness and acceptability.
3. Colour was measured using a Minolta colour meter and the results were expressed using LAB values:
L = Lightness
A = Red Colour
B = Yellow Colour

| **Sample** | **L value** | **A value** | **B value** |
|---|---|---|---|
| **Control** | 94.03 | -3.21 | 11.82 |
| **AquaMin fortified ice-cream** | 94.39 | -2.87 | 11.37 |
| **Calcium carbonate fortified ice-cream** | 95.61 | -2.84 | 10.83 |

A statistical Student's t- Test was carried out on these values (9 values for each sample) and the results of the test showed that there was a significant difference between the control and the ice-cream fortified with calcium carbonate for each of the three parameters . AquaMin only had a significant effect on the A value of the ice-cream, it did not effect the L or B values.
4. A further batch of ice-cream was made to assess if calcium addition effected the over-run properties of the ice-cream. Production conditions were kept constant and it appears that addition of calcium did not have a significant effect on the over-run properties. The control, AquaMin fortified ice-cream and the calcium carbonate fortified ice-cream had the following over-run of 130%, 139% and 136% respectively.

### EXAMPLE 7

### CALCIUM FORTIFICATION OF LOW FAT ICE-CREAM

Low fat ice-cream was made using a standard recipe.

Three batches of ice-cream were made :
1. + 0.8% AquaMin
2. + 0.6% Calcium carbonate
3. Control

### (Addition of AquaMin provides 100% Calcium fortification in a 200g serving)

Processing conditions were kept constant and it was determined that there were no differences in the over-run between the different batches.

A sensory analysis of the ice-cream was carried out under controlled conditions (report available upon request) during which panellists were asked to assess samples from the three batches ice-cream using the following parameters :
Sample:
   Sweetness (1 = not sweet, = extremely sweet)
   Creaminess (1 = not creamy, 5 = extremely creamy)
   Iciness/Coarseness (1 = very icy, 5 = not icy)
   Overall acceptability (1 = worst, 5 = best)

17 panellists took part in this analysis and the results were as follows:

| | Sample A +0.8% AquaMin | Sample B +0.6% CaCO₃ | Sample C Control |
|---|---|---|---|
| Sweetness | 3.29 | 3.12 | 3.18 |
| Creaminess | 4 | 3.35 | 3.53 |
| Iciness/Coarseness | 4.71 | 4.24 | 4.41 |
| Overall Acceptability | 3.18 | 3.29 | 3.47 |
| Preference | 7 | 3 | 7 |

A statistical Student's t- Test was carried out on the above data and this showed that there was not a significant difference in the results between sweetness and overall acceptability of the product. However differences were evident in the parameters of creaminess and iciness/coarseness. Sample A was significantly creamier and significantly less icy/coarse than Samples B and C.

### EXAMPLE 8

### CALCIUM FORTIFICATION OF SWEETS

Handmade sweets were made using a traditional recipe of:
Sugar
Water
Cream of Tartar
Flavour
Malic Acid
Food Colours

AquaMin was added to this recipe at a level of 3.5%.

The sugar and water is boiled, allowed to cool, and as it solidifies on a metal bench, the AquaMin, Malic Acid, Savour and dyes are splashed on and folded into the mixture.

It is necessary to add extra Malic Acid (AquaMin: Malic Acid, 3:1) to counteract a bland flavour.

A variety of flavours and colours were used.

Each sweet weighs approximately 3.5g and contains 40 mgs of Calcium.

## Claims

1. Use in a solid or semi-solid foodstuff, of a material to improve organoleptic and physical properties and calcium consent **characterised in that** the material is a calcareous residue of corallinaceae consisting primarily of mineral substances particularly calcium carbonate with a content of heavy metals below the upper limits acceptable for edible products.

2. A use according to claim 1 wherein the product is a foodstuff having an emulsified oil or fat phase into which is incorporated the corallinaceae residue and which has improved organoleptic properties as compared to the same product free of said residue.

3. A use according to claim 1 in which the foodstuff containing fat material in which the corallinaceae residue is distributed generally in the foodstuff.

4. A use according to claim 1 in which the foodstuff is a foodstuff to be consumed in a frozen form.

5. A use according to claim 1 in which the foodstuff is a carbohydrate product.

6. A use according to claim 3 in which the foodstuff is a chocolate product.

7. A use according to claim 5 in which the foodstuff is a farinaceous product.

8. A use according to claim 7 in which the foodstuff is primarily composed of a starch based material.

## Patentansprüche

1. Verwendung eines Materials in einem festen oder halbfesten Lebensmittel, um organoleptische und physikalische Eigenschaften zu verbessern und Calciumgehalt, **dadurch gekennzeichnet, dass** das Material ein kalkhaltiger Rückstand von Corallinaceae ist, hauptsächlich bestehend aus mineralischen Substanzen und speziell Calciumcarbonat mit einem Gehalt an Schwermetallen unterhalb der Obergrenzen, die für Speiseprodukte zulässig sind.

2. Verwendung nach Anspruch 1, worin das Produkt ein Lebensmittel ist, das über eine emulgierte Öl- oder Fettphase verfügt, in die der Corallinaceae-Rückstand eingearbeitet ist und das über verbesserte organoleptische Eigenschaften im Vergleich zu dem gleichen Produkt verfügt, das frei von diesem Rückstand ist.

3. Verwendung nach Anspruch 1, worin das Lebensmittel ein Fettmaterial enthält, in welchem der Corallinaceae-Rückstand im Allgemeinen in dem Lebensmittel verteilt ist.

4. Verwendung nach Anspruch 1, worin das Lebensmittel ein Lebensmittel ist, das in Gefrierform verbraucht wird.

5. Verwendung nach Anspruch 1, worin das Lebensmittel ein Kohlenhydrat-Produkt ist.

6. Verwendung nach Anspruch 3, worin das Lebensmittel ein Schokoladen-Produkt ist.

7. Verwendung nach Anspruch 5, worin das Lebensmittel ein mehlhaltiges Produkt ist.

8. Verwendung nach Anspruch 7, worin das Lebensmittel hauptsächlich zusammengesetzt ist aus einem Material auf Stärkebasis.

## Revendications

1. Utilisation dans un produit alimentaire solide ou semi-solide d'une matière pour améliorer les propriétés organoleptiques et physiques et la teneur en calcium, **caractérisée en ce que** la matière est un résidu calcaire de corallinacées constitué principalement par des substances minérales, en particulier du carbonate de calcium avec une teneur en métaux lourds inférieure aux limites supérieures acceptables pour des produits comestibles.

2. Utilisation selon la revendication 1, dans laquelle le produit est un produit alimentaire ayant une phase d'huile émulsionnée ou de graisse dans laquelle est incorporé le résidu de corallinacées, et qui possède des propriétés organoleptiques améliorées lorsqu'il est comparé au même produit exempt dudit résidu.

3. Utilisation selon la revendication 1, dans laquelle le produit alimentaire contient de la matière grasse dans laquelle le résidu de corallinacées est généralement distribué dans le produit alimentaire.

4. Utilisation selon la revendication 1, dans laquelle le produit alimentaire est un produit alimentaire destiné à être consommé sous forme congelée.

5. Utilisation selon la revendication 1, dans laquelle le produit alimentaire est un produit d'hydrate de carbone.

6. Utilisation selon la revendication 3, dans laquelle le produit alimentaire est un produit au chocolat.

7. Utilisation selon la revendication 5, dans laquelle le produit alimentaire est un produit farineux.

8. Utilisation selon la revendication 7, dans laquelle le produit alimentaire est principalement composé d'une matière à base d'amidon.
